# FASCICULE DE BREVET EUROPEEN

(11) **EP 4 153 126 B1**
(45) Date de publication et mention de la délivrance du brevet: **10.07.2024**
(21) Numéro de dépôt: 21732474.8
(22) Date de dépôt: 18.05.2021
(51) Int. Cl.: A61K 8/60, A61K 8/67, A61K 8/9789, A61Q 3/00, A61Q 3/02, A61K 8/73

(54) **COMPOSITION COSMÉTIQUE DE SOIN POUR LES ONGLES SOUS LA FORME D'UN VERNIS**
KOSMETISCHE PFLEGEZUSAMMENSETZUNG FÜR NÄGEL IN FORM EINES LACKS
COSMETIC CARE COMPOSITION FOR NAILS IN THE FORM OF A VARNISH

(30) Priorité: 19.05.2020 FR 2005017
(43) Date de publication de la demande: 29.03.2023
(73) Titulaire: Fiabila, 28130 Maintenon (FR)
(72) Inventeur: PAILOT, Arnaud, 55230 SPINCOURT (FR); RENARD, Christine, 28130 MAINTENON (FR); ROUF, Charlotte, 77700 BAILLY-ROMAINVILLIERS (FR)
(74) Mandataire: Ipsilon
(86) Numéro de dépôt international: PCT/FR2021/050863
(87) Numéro de publication internationale: WO 2021/234270

(56) Documents cités:
- WO-A1-2007/024915
- FR-A1- 2 854 800
- FR-A1- 2 896 154
- FR-A1- 2 952 302
- JP-A- H10 226 633
- JOUANDEAUD M ET AL: "Barrier function and dry skin: Use of rhamnogalacturonans as new therapeutic strategy", CAPLUS, 9 July 2004 (2004-07-09), XP002399230

## Description

La présente invention concerne le domaine des compositions cosmétiques pour les ongles, plus particulièrement le domaine des compositions cosmétiques se présentant sous la forme d'un vernis, et utilisation de ces compositions cosmétiques pour améliorer l'état de surface des ongles.

### ETAT DE LA TECHNIQUE

Les formulateurs de vernis à ongles cherchent depuis longtemps à incorporer dans les dits vernis des substances ou molécules pouvant améliorer l'état de surface des ongles.

Ainsi pour renforcer ou durcir les ongles fragilisés, cassants, dédoublés ou fissurés il a été proposé des compositions de vernis comprenant des aldéhydes ou des acides.

Cependant l'incorporation de formaldéhyde, connue depuis longtemps, est maintenant écartée en raison de sa toxicité et de son caractère allergisant. Il en est de même pour l'utilisation du glyoxal (décrit dans le brevet GB 2 196 978), du citral décrit comme durcisseur dans le brevet EP 1 408 917, également allergisant.

Quant aux acides, tels que par exemple les acides alpha-hydroxylés, ils provoquent souvent des irritations de la peau entourant l'ongle, et sont en outre difficiles à stabiliser dans les compositions de vernis.

La tendance actuelle est également de rechercher à utiliser des substances d'origine naturelle, plutôt que des molécules synthétiques. Cependant ces substances doivent être compatibles avec les milieux solvantés organiques ou aqueux des vernis à ongles. De plus ils ne doivent pas impacter défavorablement les qualités de ces vernis.

### BUTS DE L'INVENTION

Un premier but de l'invention est donc de proposer une composition de soin pour les ongles se présentant sous la forme d'un vernis renfermant un actif d'origine naturelle, ne présentant pas les inconvénients mentionnés ci-dessus.

Un autre but de l'invention est de proposer une composition de soin pour les ongles se présentant sous la forme d'un vernis qui soit stable au cours du temps, aux concentrations de l'actif auxquelles ce dernier est efficace en tant que soin pour les ongles, y compris pour différentes teintes de vernis. Par soin pour les ongles on entend en particulier une amélioration de l'état de la surface de l'ongle.

Un autre but de l'invention est de proposer une composition de soin pour les ongles se présentant sous la forme d'un vernis renfermant un actif d'origine naturelle ne dégradant pas les propriétés de brillance, d'adhérence, de dureté et de stabilité du vernis de base (c'est-à-dire d'un vernis ne renfermant pas ledit actif).

### DESCRIPTION DE L'INVENTION

Dans la recherche d'une substance ou molécule pouvant pallier les inconvénients ci-dessus, les inventeurs ont découvert, de manière surprenante, que l'incorporation, dans une composition de vernis à ongles de rhamnogalacturonane et d'acides uronique, notamment issus d'un extrait de châtaigne pouvait répondre aux buts invoqués précédemment.

A cet effet, la présente invention propose une composition cosmétique de soin pour les ongles se présentant sous la forme d'un vernis à appliquer à la surface de l'ongle, caractérisée en ce qu'elle renferme au moins un rhamnogalacturonane et au moins un acide uronique.

La composition cosmétique de soin pour les ongles peut renfermer un extrait de châtaigne, de préférence *Castanea sativa,* comprenant des rhamnogalacturonanes et des acides uroniques.

De préférence, ladite composition comprend une quantité d'extrait de châtaigne comprise entre 0,01 % et 10 % en poids, de préférence comprise entre 0,05 % et 8 % en poids, de préférence comprise entre 0,1 % et 6 % en poids.

L'extrait de châtaigne est de préférence un extrait aqueux préparé selon le procédé décrit dans le brevet français FR 2 854 800 et commercialisé sous le nom Recoverine^{®} par la société SILAB et renfermant entre 8 % et 12 % massique de matières sèches. Cet extrait comprend notamment des rhamnogalacturonanes et des acides uroniques. Il peut être mis en oeuvre dans la composition selon l'invention sous forme aqueuse ou sous forme sèche.

Cet actif de soin s'est révélé particulièrement intéressant, en agissant sur les kératinocytes de l'ongle, pour améliorer l'état de surface de l'ongle, en particulier pour nourrir et hydrater l'ongle, sans provoquer d'irritations de la peau chez les sujets testés, ni générer d'autres effets secondaires néfastes.

Il s'avère également que cet extrait aqueux de châtaigne peut être incorporé soit dans les vernis solvantés, c'est-à-dire dans des vernis comprenant un milieu solvantaire majoritairement (plus de 50% massique) formé de solvants organiques, soit dans des vernis aqueux, c'est-à-dire dont le milieu solvantaire comprend majoritairement (plus de 50% massique) de l'eau.

Selon un premier mode de réalisation de l'invention, la composition de soin pour les ongles est caractérisée en ce que le vernis est un vernis solvanté comprenant un milieu solvantaire organique, au moins un agent filmogène, au moins un plastifiant, au moins une résine, et éventuellement un agent de rhéologie et renferme ledit extrait de châtaigne dans une proportion comprise entre 0,01 % et 1 % en poids, de préférence comprise entre 0,05 % et 0,8 % en poids, de préférence encore comprise entre 0,1 % et 0,6 % en poids.

Il est apparu qu'à de telles concentrations l'extrait de châtaigne n'affecte pas la stabilité du vernis par rapport à un vernis sans extrait de châtaigne, en particulier, comme il sera vu dans les exemples, ni la sédimentation, ni la synérèse du vernis.

Le milieu solvantaire organique peut comprendre un ou plusieurs solvants choisi(s) parmi : les esters à chaîne courte ayant de 3 à 8 atomes de carbone au total, tels que l'acétate d'éthyle, l'acétate de méthyle, l'acétate de propyle, l'acétate d'isopropyle, l'acétate de butyle, l'acétate d'isopentyle, l'acétate de méthoxypropyle, le lactate de butyle, les cétones liquide à température ambiante, les alcools tels que l'éthanol, l'isopropanol, le butanol, le diacétone alcool, les hydrocarbures aliphatiques linéaires ou ramifiés, saturés, tels que les alcanes, ou insaturés, liquides à température ambiante, les hydrocarbures aromatiques, tels que le toluène ou le xylène, et leurs mélanges.

De préférence, le milieu solvantaire organique comprend l'acétate d'éthyle et/ou l'acétate de butyle et/ou au moins un alcool choisi parmi l'éthanol, l'isopropanol et le butanol.

Selon un second mode de réalisation de l'invention, la composition est caractérisée en ce que le vernis est un vernis à base aqueuse comprenant un milieu solvantaire aqueux, au moins un agent filmogène, au moins un plastifiant, au moins une résine, éventuellement un agent de rhéologie et renferme ledit extrait de châtaigne dans une proportion comprise entre 0,01 % et 10 % en poids, de préférence comprise entre 0,05 % et 8 % en poids, de préférence encore comprise entre 0,1 % et 6 % en poids.

Selon la présente invention, le vernis comprend au moins un agent filmogène qui est de préférence un agent filmogène cellulosique, choisi parmi la nitrocellulose, la carboxyméthylcellulose, l'hydroxyéthylcellulose, l'hydroxypropylcellulose, l'acétobutyrate de cellulose et/ou l'acétopropionate de cellulose.

Le vernis peut également comprendre un ou plusieurs monomères ou oligomères à base (méth)acrylate, uréthane, carbamate, isophorones et/ou isocyanures, et un ou plusieurs photoinitiateurs.

De plus ledit vernis peut comprendre le vernis comprend au moins une résine choisie parmi les résines polyvinylbutyral, résines shellac, résines élémi, résines alkydes, résines polyesters ; résines epoxytosylamide ; résines époxy ; résines acrylates ; résines styrène-acrylates ; résines polyuréthane ; le sucrose de benzoate ; résines tosylamide/formaldéhyde, résines toluène sulfonamide/formaldéhyde, ou des polymères et/ou copolymères de ces différentes résines.

L'ajout dudit extrait aqueux de châtaigne est apparu intéressant pour les vernis teintés, renfermant au moins une substance colorante, des pigments, des nacres, des paillettes, des particules métalliques ou un mélange de ceux-ci. Il est apparu que la stabilité de ces vernis est maintenue dans le temps. Néanmoins selon une variante de l'invention, le vernis est un vernis incolore, ne refermant ni pigment, ni substance colorante.

De manière avantageuse, la composition cosmétique selon l'invention peut aussi comprendre des produits de soin de l'ongle autres que l'extrait de châtaigne choisi(s) parmi les vitamines A, B, C, E et leurs dérivés, du calcium, des silanes, des alkylsulfones, des acides aminés ou peptides, notamment ceux contenant du soufre, et leurs sels, de la kératine hydrolysée, des alpha-hydroxyacides, de l'urée et ses dérivés, des huiles d'origine végétale, des extraits de végétaux autres que l'extrait de châtaigne, et une combinaison de ceux-ci.

La présente invention concerne également l'utilisation de la composition cosmétique décrite ci-dessus pour améliorer l'état de surface de l'ongle, en particulier pour nourrir et hydrater l'ongle. L'utilisation de cette composition cosmétique de soin est une utilisation non thérapeutique.

L'avantage d'un vernis, par rapport à une crème qui nécessite d'être appliquée quotidiennement, est que son action est plus durable dans le temps, le vernis pouvant rester sur l'ongle plusieurs jours sans être éliminé, notamment lors du lavage des mains.

### EXEMPLES

Les exemples suivants permettent d'illustrer, de manière non limitative, la présente invention. Dans l'ensemble des formulations, la concentration des différents constituants est exprimée en % en poids du poids total de la composition cosmétique.

### Test effectués

Différentes compositions cosmétiques sous la forme de vernis à ongle de type vernis à base solvant organique ou de type vernis aqueux ont été préparées et appliquées sur des ongles. Les tests ci-après ont été réalisés sur ces vernis :
Brillance : Sur une plaque de type Leneta, 100µm de vernis a été appliqué. Après séchage, la brillance a été mesurée à un angle d'indice de 60°, au moyen d'un Brillancemètre MINOLTA 268.

Flexibilité : Sur une plaque en aluminium recouverte d'un vernis de 300 µm humide, on réalise un emboutissage lent et on mesure la profondeur de la déformation de la plaque de métal (ISO1520) (exprimée en mm)

Dureté du vernis : a été mesurée à l'aide d'un pendule de Persoz sur une plaque de verre recouverte d'un vernis d'épaisseur de 100µm humide, après séchage à température ambiante, selon la norme ISO1522 (exprimée en s).

Adhérence : un « Cross hatch test » est réalisé sur plaque de verre. La note 0 correspond à une absence de perte d'adhésion. La note 5 correspond à une perte totale d'adhésion.

Extrait sec : Dans une coupelle, on verse entre 0,5 et 1g de vernis. Cette plaque est ensuite mise à l'étuve durant 3 heures à 100°C, puis pesée pour calculer l'extrait sec du vernis.

Stabilité : Les vernis sont conditionnés dans des flacons en verre et stockés dans une étuve à 50°C durant 1 mois. La couleur du vernis est ensuite comparée à un vernis de référence ne contenant pas l'actif.

Viscosité : La viscosité du produit est mesurée à 25°C à l'aide d'un viscosimètre Brookfield. Les mesures sont prises après une rotation d'une minute à 6 tr/min (Visco 1), puis après une minute à 60 tr/min (Visco 2), enfin après une minute à 6 tr/min (Visco 3). Les valeurs sont données en mPa.s.

Teneur en eau : Le dosage de l'eau contenue dans une formule est réalisé par la méthode de Karl Fischer en utilisant un équipement Metrohm de type 915 KF Ti - Touch et des solutions de dosage commerciales.

Séchage : Après avoir appliqué un film de vernis d'épaisseur 100 µm sur une carte LENETA WDX, la carte est placée sur une plaque thermostatée (35°C ± 0,5°C). Le temps de séchage est mesuré grâce à un enregistreur circulaire placé sur le film. Cet enregistreur possède une tige en mouvement décrivant un cercle et laissant un tracé sur le film tant que celui-ci n'est pas sec. La distance angulaire laissée par la trace est ensuite convertie en temps grâce à une table de conversion préétablie.

Stabilité au jaunissement : Les produits conditionnés dans des flacons de vernis en verre sont laissés pendant un mois dans une étuve à 50°C et à température ambiante (20 °C) pour une étude comparative de la stabilité. Cette stabilité est évaluée de manière visuelle (couleur/jaunissement). Les teintes peu chargées en pigment sont particulièrement adaptées à ce type de suivi.

Stabilité à la synérèse : Les produits conditionnés dans des flacons de vernis en verre sont laissés pendant un mois dans une étuve à 50°C et à température ambiante pour une étude comparative de la stabilité. Cette stabilité est évaluée de manière visuelle (relargage de solvant laissant un cylindre à la surface du vernis). La teinte violette (voir ci-dessous) est particulièrement adaptée à ce type de suivi.

Stabilité à la sédimentation : Les produits conditionnés dans des flacons de vernis en verre sont laissés pendant un mois dans une étuve à 50°C et à température ambiante (20 °C) pour une étude comparative de la stabilité. Cette stabilité est évaluée de manière visuelle (présence de particules au fond du flacon). La teinte nacrée est particulièrement adaptée à ce type de suivi.

### Formules testées :

Les compositions de base avec agent rhéologique ont été préparées selon les formulations présentées dans le [Table 1].

**[Table 1]**

| | **Base sans actif** | **Base + 0,3% RECOVERINE^{®}** | **Base + 0,5% RECOVERINE^{®}** |
|---|---|---|---|
| **Acétate de butyle** | 42,57% | 42,27% | 42,07% |
| **Acétate d'éthyle** | 20,60% | 20,60% | 20,60% |
| **Nitrocellulose** | 14,00% | 14,00% | 14,00% |
| **Résine polyester** | 9,50% | 9,50% | 9,50% |
| **Acétyltributyl citrate** | 6,00% | 6,00% | 6,00% |
| **Alcool isopropylique** | 6,00% | 6,00% | 6,00% |
| **Stéaralkonium bentonite** | 1,30% | 1,30% | 1,30% |
| **Acide phosphorique** | 0,03% | 0,03% | 0,03% |
| **RECOVERINE^{®}** | 0,00% | 0,30% | 0,50% |

La résine polyester est un copolymère d'acide adipique / néopentyl glycol / anhydride triméllitique dilué à 70% dans l'acétate de butyle.

La nitrocellulose est à 70% dans l'isopropanol.

L'extrait aqueux de châtaigne RECOVERINE^{®} comprend environ 10 % de matière sèche active composée majoritairement de rhamnogalacturonanes et d'acides uroniques.

### Teintes

Les compositions cosmétiques selon l'invention ont été testées selon différentes teintes, au moyen de colorants et/ou de pigments (seuls ou en mélange avec du dioxyde de titane) ajoutés à la composition de base ci-dessus, avec ou sans extrait de châtaigne :
Teinte nacrée : teinte contenant une solution colorante et de la nacre à environ 1,5 %. L'inspection visuelle des nacres permet de savoir si le produit est plus sensible à la sédimentation (en comparaison avec une référence qui est ici la même composition de vernis, sans Recoverine^{®}).
Teinte violette : teinte contenant 2 % d'un mélange de pigments (Dioxyde de titane, Red 34, Bleu ferri ferrocyanide) permettant de vérifier la stabilité du produit notamment par rapport au relargage de solvant.
Teinte rose : teinte contenant un mélange de pigments (Dioxyde de titane, Red 6). Ce type de teinte faiblement chargée en pigments (0,8 %) est classiquement sensible à la synérèse et au jaunissement.
Teinte rouge : teinte contenant 2 % d'un mélange de pigments majoritaire en Red 6 et du dioxyde de titane en faible quantité.
Teinte beige : teinte très peu chargée en pigment (0,15 %) (Dioxyde de titane et oxyde de fer rouge), permettant une application discrète tout en apportant un soin renforçateur de l'ongle. Cette teinte permet d'observer une éventuelle sensibilité au jaunissement.

### Exemple 1 : stabilité après 1 mois

Différentes compositions teintées ont été préparées et observées après 1 mois à température ambiante (20°C) ainsi qu'à 50°C. Les résultats sont présentés dans le [Table 2] ci-après.

**[Table 2]**

| **Conditions de stockage** | | **Température ambiante (20°C)** | **50° C** |
|---|---|---|---|
| **Teinte rose** | **0,0% RECOVERINE^{®}** | RAS | Légère synérèse |
| | **0,3% RECOVERINE^{®}** | RAS | Légère synérèse |
| | **0,5% RECOVERINE^{®}** | RAS | Légère synérèse |
| **Teinte violette** | **0,0% RECOVERINE^{®}** | RAS | Absence de synérèse |
| | **0,3% RECOVERINE^{®}** | RAS | Absence de synérèse |
| | **0,5% RECOVERINE^{®}** | RAS | Légère synérèse |
| **Teinte nacrée** | **0,0% RECOVERINE^{®}** | RAS | Absence de sédimentation |
| | **0,3% RECOVERINE^{®}** | RAS | Absence de sédimentation |
| | **0,5% RECOVERINE^{®}** | RAS | Absence de sédimentation |
| **Teinte beige** | **0,0%** | RAS | Pas jaunissement |
| | **0,5%** | RAS | Pas de jaunissement |

La teinte beige a été utilisée pour prouver via le panel test (voir plus loin l'exemple 5) que l'ajout d'actif dans le vernis apporte un réel bénéfice pour l'ongle et pour suivre un éventuel phénomène de jaunissement au cours du temps.

Ces résultats nous montrent que la présence de l'extrait de châtaigne n'altère pas la stabilité des teintes même si elles sont soumises à des conditions extrêmes puisque les comportements avec 0,3% et 0,5% en extrait de châtaigne sont similaires aux résultats obtenus avec les teintes sans actifs.

La teinte violette, considérée comme une teinte généralement critique pour évaluer la stabilité des produits vis-à-vis de la synérèse, montre une légère synérèse à 50°C quand 0,5% de RECOVERINE^{®} sont ajoutés. Ce comportement montre que 0,5% semble être la concentration limite d'utilisation de cet extrait aqueux dans les vernis solvantaires ayant des propriétés thixotropes. Ce phénomène est dû à la présence d'eau dans l'actif tel que fourni par la société SILAB : en effet cette eau favorise un indice de thixotropie plus élevé en jouant le rôle d'activateur de l'agent thixotropant.

### Exemple 2 : rhéologie

Après un mois dans une étuve à l'obscurité et à une température de 50 °C, les résultats obtenus sur trois compositions teintées différentes (rose, violette sensible à la synérèse, rouge et rouge nacrée sensible à la sédimentation) préparées selon la composition de base de l'exemple 1 (avec 2% de pigment sont présentés dans le [Table 3] :

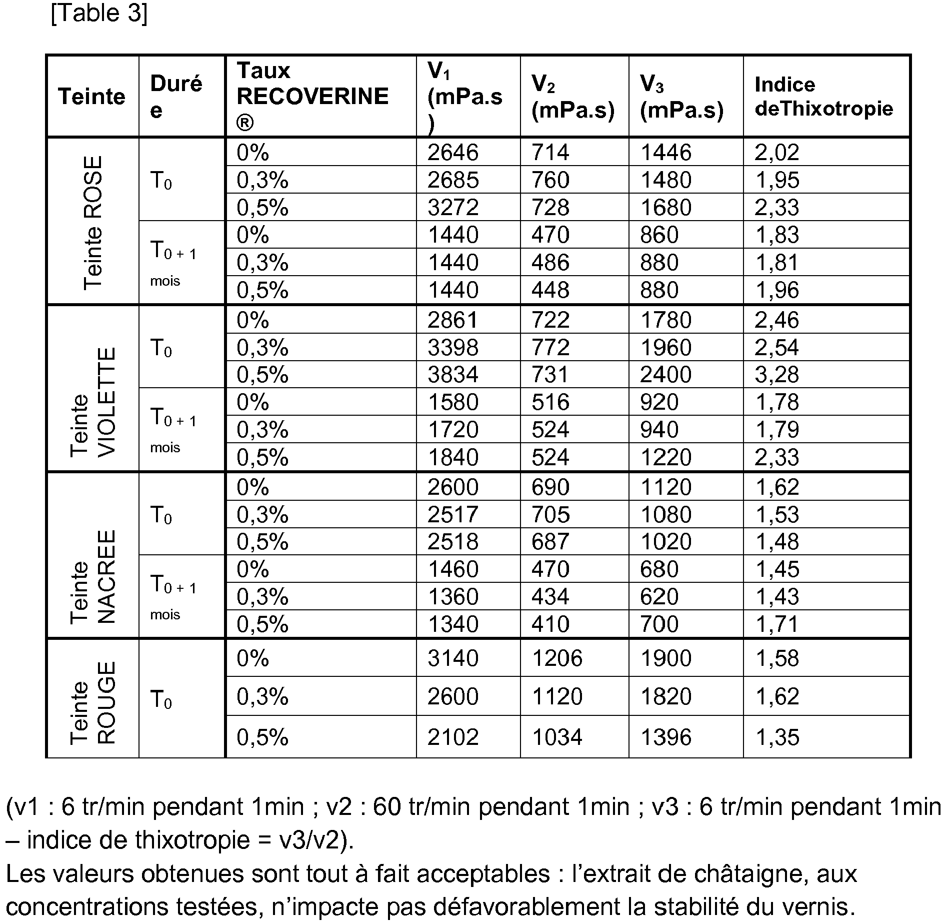

### Exemple 3 : propriétés générales

Dans cet exemple sont présentées l'ensemble des propriétés testées en comparant une composition selon l'exemple 1 teintée (teinte rouge - renfermant 2% massique de pigment) sans Recoverine^{®} et avec 0,3% de Recoverine^{®}.

L'étude a également été réalisée sur la composition de base seule (sans ajout de pigment) avec 0,3% ou sans Recoverine^{®} : voir [Table 4]

**[Table 4]**

| | Base + 0% Recoverine^{®} | | Base + 0,3% Recoverine^{®} | |
|---|---|---|---|---|
| | Base seule | Teinte rouge | Base seule | Teinte rouge |
| Visco 1 (mPa.s) | 2420 | 3140 | 4100 | 2600 |
| Visco 2 (mPa.s) | 916 | 1206 | 1090 | 1120 |
| Visco 3 (mPa.s) | 1660 | 1900 | 2920 | 1820 |
| Indice thixo | 1,81 | 1,58 | 2,68 | 1,63 |
| Brillance (%)⁽¹⁾ | 84,1 | 83,3 | 84,0 | 82,6 |
| Dureté (s) ⁽¹⁾ | 198 | 199 | 191 | 208 |
| Adhérence | 0 | 0 | 0 | 0 |
| Extrait Sec (%) | 31,58 | *Nd* | 31,32 | *Nd* |
| Teneur en eau (%) | 0,64 | *Nd* | 0,74 | *Nd* |
| Séchage (minute) | 2'30 | 3'30 | 3'15 | 3'00 |
| Emboutissage (mm) | 4,70 | 4,45 | 5,20 | 4,45 |
| Pliage | OK | Légères fissures | OK | Légères fissures |

| | | | | |
|---|---|---|---|---|
| *(1)valeur moyennée sur 3 points* - *Nd : non déterminé* | | | | |

Cette étude comparative nous montre que la seule différence notable se situe au niveau de la composition de base seule avec ou sans l'extrait de châtaigne. Ce phénomène d'augmentation de la rhéologie (viscosité/thixotropie) est dû à la présence d'eau dans cet extrait aqueux, comme indiqué précédemment. L'eau est connue pour être un très bon activateur des argiles (utilisées pour réguler la rhéologie des vernis à ongles) mais son action n'est pas durable dans le temps. On peut d'ailleurs remarquer que l'ajout de pigments réduit considérablement l'action de l'eau sur les argiles.

Pour tous les autres paramètres mesurés, les résultats sont satisfaisants, l'extrait de châtaigne dans ces compositions de vernis n'impacte pas négativement les propriétés.

### Exemple 4 : vernis aqueux

L'extrait de châtaigne RECOVERINE^{®} étant un extrait aqueux, il peut tout à fait être incorporé à un vernis à base aqueuse. Les formulations peuvent être par exemple dans les gammes de concentrations massiques suivantes :
H₂O 50,0 - 90,0%
Carbomer filmogene viscosant 0,1 - 2,0%
Trisodium phosphate 2,0 - 4,0%
Glycérine 5,0 - 15,0%
Propylene glycol 1,0 - 10%
Phenoxyethanol 0,5 - 1,5%
Recoverine^{®} 0,1 % - 10,0%

Un test réalisé avec une composition de vernis aqueux à 3 % de Recoverine^{®} appliquée sur les ongles deux fois par semaine pendant 15 jours a montré une amélioration notable de la qualité des ongles : les ongles étaient plus hydratés, moins cassants.

### Exemple 5 : test auprès de consommateurs

Un test d'usage a été réalisé sur un panel de 15 volontaires, pendant 2 semaines. La composition de vernis solvantée (conforme à celle de l'exemple 1 avec 0,15 % de pigments - teinte beige) renfermant 0,5 % de RECOVERINE^{®} a été appliquée deux fois par semaine sur les ongles de tous les doigts en deux couches. Chaque paneliste a évalué les propriétés à J0, à 7jours (J7) et à 14 jours (J14). Elle a été comparée à des compositions de vernis renfermant soit des acides alpha-hydroxylés, soit un mélange d'acides alpha hydroxylés et d'acides polyhydroxylés connues pour améliorer l'état des matières kératiniques.

A J0 et J14 les panelistes ont attribué une notre de 1 à 10 sur la qualité de leurs ongles avant et après traitement. Les panelistes ont noté (p<0,05) une amélioration notable de l'état des ongles, plus particulièrement :
Ongles moins fragiles → 87% des personnes
Ongles moins cassants → 87% des personnes
Ongles qui se dédoublent moins → 87% des personnes
Ongles moins mous → 80% des personnes
Ongles mieux hydratés → 80% des personnes
Ongles moins rugueux → 80% des personnes.

Aucune personne n'a noté de difficulté d'application de la composition de vernis, ni de réduction de la brillance. Seul le temps de séchage sur l'ongle était légèrement plus long.

Néanmoins, la composition testée améliore, de manière surprenante, l'hydratation des ongles, dès le 7^{e} jour d'application.

## Revendications

1. Composition cosmétique de soin pour les ongles se présentant sous la forme d'un vernis à appliquer à la surface de l'ongle, **caractérisée en ce qu'**elle renferme au moins un rhamnogalacturonane et au moins un acide uronique.

2. Composition selon la revendication 1 **caractérisée en ce qu'**elle renferme un extrait de châtaigne, de préférence de *Castanea sativa,* comprenant des rhamnogalacturonanes et des acides uroniques.

3. Composition selon la revendication 2, **caractérisée en ce qu'**elle comprend une quantité d'extrait de châtaigne comprise entre 0,01 % et 10 % en poids, de préférence comprise entre 0,05 % et 8% en poids, de préférence comprise entre 0,1 % et 6 % en poids.

4. Composition selon l'une quelconque des revendications 2 ou 3, **caractérisée en ce que** le vernis est un vernis solvanté comprenant un milieu solvantaire organique, au moins un agent filmogène, au moins un plastifiant, au moins une résine, éventuellement un agent de rhéologie, et renferme ledit extrait de châtaigne dans une proportion comprise entre 0,01 % et 1 % en poids, de préférence comprise entre 0,05 % et 0,8 % en poids, de préférence encore comprise entre 0,1 % et 0,6 % en poids.

5. Composition selon la revendication 4, **caractérisée en ce que** le milieu solvantaire organique comprend un ou plusieurs solvants choisi(s) parmi : les esters à chaîne courte ayant de 3 à 8 atomes de carbone au total, tels que l'acétate d'éthyle, l'acétate de méthyle, l'acétate de propyle, l'acétate d'isopropyle, l'acétate de butyle, l'acétate d'isopentyle, l'acétate de méthoxypropyle, le lactate de butyle, les cétones liquide à température ambiante, les alcools tels que l'éthanol, l'isopropanol, le butanol, le diacétone alcool, les hydrocarbures aliphatiques linéaires ou ramifiés, saturés, tels que les alcanes, ou insaturés, liquides à température ambiante, les hydrocarbures aromatiques, tels que le toluène ou le xylène, et leurs mélanges.

6. Composition selon la revendication 5, **caractérisée en ce que** le milieu solvantaire organique comprend l'acétate d'éthyle et/ou l'acétate de butyle et/ou au moins un alcool choisi parmi l'éthanol, l'isopropanol et le butanol.

7. Composition selon l'une quelconque des revendications 2 ou 3, **caractérisée en ce que** le vernis est un vernis à base aqueuse comprenant un milieu solvantaire aqueux, au moins un agent filmogène, au moins un plastifiant, au moins une résine, éventuellement un agent de rhéologie et renferme ledit extrait de châtaigne dans une proportion comprise entre 0,01 % et 10 % en poids, de préférence comprise entre 0,05 % et 8 % en poids, de préférence encore comprise entre 0,1 % et 6 % en poids.

8. Composition selon l'une quelconque des revendications précédentes, **caractérisée en ce que** le vernis comprend au moins un agent filmogène cellulosique, choisi parmi la nitrocellulose, la carboxyméthylcellulose, l'hydroxyéthylcellulose, l'hydroxypropylcellulose, l'acétobutyrate de cellulose et/ou l'acétopropionate de cellulose.

9. Composition selon l'une quelconque des revendications précédentes, **caractérisée en ce que** le vernis comprend un ou plusieurs monomères ou oligomères à base (méth)acrylate, uréthane, carbamate, isophorones et/ou isocyanures, et un ou plusieurs photoinitiateurs.

10. Composition selon l'une quelconque des revendications 4 à 9, **caractérisée en ce que** le vernis comprend au moins une résine choisie parmi les résines polyvinylbutyral, résines shellac, résines élémi, résines alkydes, résines polyesters ; résines epoxytosylamide ; résines époxy ; résines acrylates ; résines styrène-acrylates ; résines polyuréthane ; le benzoate de sucrose; résines tosylamide/formaldéhyde, résines toluènesulfonamide/formaldéhyde, ou des polymères et/ou copolymères de ces différentes résines.

11. Composition selon l'une quelconque des revendications précédentes, **caractérisée en ce que** le vernis est un vernis teinté, renfermant au moins une substance colorante, des pigments, des nacres, des paillettes, des particules métalliques ou un mélange de ceux-ci.

12. Composition selon l'une quelconque des revendications précédentes, **caractérisée en ce qu'**elle comprend un ou plusieurs produits de soin de l'ongle autres que l'extrait de châtaigne choisi(s) parmi les vitamines A, B, C, E et leurs dérivés, du calcium, des silanes, des alkylsulfones, des acides aminés ou peptides, notamment ceux contenant du soufre, et leurs sels, de la kératine hydrolysée, des alpha-hydroxyacides, de l'urée et ses dérivés, des huiles d'origine végétale, des extraits de végétaux autres que l'extrait de châtaigne, et une combinaison de ceux-ci.

13. Utilisation de la composition cosmétique selon l'une quelconque des revendications précédentes pour améliorer l'état de surface de l'ongle.

14. Utilisation de la composition selon la revendication 13 pour nourrir et hydrater l'ongle.

## Patentansprüche

1. Kosmetische Zusammensetzung zur Pflege der Nägel, die in Form eines Lacks vorliegt, der auf die Oberfläche des Nagels aufgetragen wird, **dadurch gekennzeichnet, dass** sie mindestens ein Rhamnogalacturonan und mindestens eine Uransäure enthält.

2. Zusammensetzung nach Anspruch 1, **dadurch gekennzeichnet, dass** sie einen Kastanienextrakt, vorzugsweise von Castanea sativa, enthält, umfassend Rhamnogalacturonane und Uransäuren.

3. Zusammensetzung nach Anspruch 2, **dadurch gekennzeichnet, dass** sie eine Menge an Kastanienextrakt zwischen 0,01 Gewichts-% und 10 Gewichts-%, vorzugsweise zwischen 0,05 Gewichts-% und 8 Gewichts-%, vorzugsweise zwischen 0,1 Gewichts-% und 6 Gewichts-%, enthält.

4. Zusammensetzung nach einem der Ansprüche 2 oder 3, **dadurch gekennzeichnet, dass** der Lack ein lösungsmittelhaltiger Lack ist, umfassend ein organisches Lösungsmittelmedium, mindestens einen Filmbildner, mindestens einen Weichmacher, mindestens ein Harz, optional ein Rheologiemittel und den Kastanienextrakt in einem Anteil von 0,01 bis 1 Gewichts-%, vorzugsweise von 0,05 bis 0,8 Gewichts-%, bevorzugter von 0,1 bis 0,6 Gewichts-%, enthält.

5. Zusammensetzung nach Anspruch 4, **dadurch gekennzeichnet, dass** das organische Lösungsmittelmedium ein oder mehrere Lösungsmittel umfasst, die ausgewählt sind aus: kurzkettigen Estern, die insgesamt 3 bis 8 Kohlenstoffatome aufweisen, wie beispielsweise Ethylacetat, Methylacetat, Propylacetat, Isopropylacetat, Butylacetat, Isopentylacetat, Methoxypropylacetat, Butyllactat, bei Raumtemperatur flüssige Ketone, Alkohole, wie beispielsweise Ethanol, Isopropanol, Butanol, Diacetonalkohol, geradkettige oder verzweigte, gesättigte aliphatische Kohlenwasserstoffe, wie beispielsweise Alkane oder ungesättigte Kohlenwasserstoffe, die bei Raumtemperatur flüssig sind, aromatische Kohlenwasserstoffe, wie beispielsweise Toluol oder Xylol und deren Gemische.

6. Zusammensetzung nach Anspruch 5, **dadurch gekennzeichnet, dass** das organische Lösungsmittelmedium Ethylacetat und/oder Butylacetat und/oder mindestens einen Alkohol, ausgewählt aus Ethanol, Isopropanol und Butanol, umfasst.

7. Zusammensetzung nach einem der Ansprüche 2 oder 3, **dadurch gekennzeichnet, dass** der Lack ein Lack auf Wasserbasis ist, umfassend ein wässriges Lösungsmittelmedium, mindestens einen Filmbildner, mindestens einen Weichmacher, mindestens ein Harz, optional ein Rheologiemittel und den Kastanienextrakt in einem Anteil von 0,01 bis 10 Gewichts-%, vorzugsweise von 0,05 bis 8 Gewichts-%, bevorzugter von 0,1 bis 6 Gewichts-%, enthält.

8. Zusammensetzung nach einem der vorherigen Ansprüche, **dadurch gekennzeichnet, dass** der Lack mindestens einen Cellulose-Filmbildner umfasst, ausgewählt aus Nitrocellulose, Carboxymethylcellulose, Hydroxyethylcellulose, Hydroxypropylcellulose, Celluloseacetobutyrat und/oder Celluloseacetopropionat.

9. Zusammensetzung nach einem der vorherigen Ansprüche, **dadurch gekennzeichnet, dass** der Lack ein oder mehrere Monomere oder Oligomere auf (Meth)acrylat-, Urethan-, Carbamat-, Isophoron- und/oder Isocyanidbasis und einen oder mehrere Photoinitiatoren umfasst.

10. Zusammensetzung nach einem der Ansprüche 4 bis 9, **dadurch gekennzeichnet, dass** der Lack mindestens ein Harz umfasst, das ausgewählt ist aus Polyvinylbutyralharzen, Schellackharzen, Elemiharzen, Alkydharzen, Polyesterharzen; Epoxytosylamidharzen; Epoxidharzen; Acrylatharzen; Styrol-Acrylatharzen; Polyurethanharzen; Sucrasebenzoat; Tosylamid/Formaldehydharzen, Toluolsulfonamid/Formaldehydharzen, oder Polymeren und/oder Copolymeren dieser verschiedenen Harze.

11. Zusammensetzung nach einem der vorherigen Ansprüche, **dadurch gekennzeichnet, dass** der Lack ein getönter Lack ist, der mindestens einen Farbstoff, Pigmente, Perlmutt, Flitter, Metallpartikel oder ein Gemisch davon enthält.

12. Zusammensetzung nach einem der vorherigen Ansprüche, **dadurch gekennzeichnet, dass** sie ein oder mehrere andere Nagelpflegeprodukte als Kastanienextrakt umfasst, die ausgewählt sind aus den Vitaminen A, B, C, E und ihren Derivaten, Calcium, Silanen, Alkylsulfonen, Aminosäuren oder Peptiden, insbesondere schwefelhaltigen, und ihren Salzen, hydrolysiertem Keratin, Alpha-Hydroxysäuren, Harnstoff und seinen Derivaten, Ölen pflanzlichen Ursprungs, anderen Pflanzenextrakten als Kastanienextrakt und einer Kombination davon.

13. Verwendung der kosmetischen Zusammensetzung nach einem der vorherigen Ansprüche zum Verbessern des Oberflächenzustands des Nagels.

14. Verwendung der Zusammensetzung nach Anspruch 13, um den Nagel zu nähren und zu hydratisieren.

## Claims

1. A cosmetic nail care composition in the form of a varnish to be applied to the surface of the nail, **characterized in that** same contains at least one rhamnogalacturonan and at least one uronic acid.

2. The composition according to claim 1, **characterized in that** same contains an extract of chestnut, preferably of Castanea sativa, comprising rhamnogalacturonans and uronic acids.

3. The composition according to claim 2, **characterized in that** same comprises a quantity of chestnut extract of between 0.01% and 10% by weight, preferably between 0.05% and 8% by weight, preferably between 0.1% and 6% by weight.

4. The composition according to any of claims 2 or 3, **characterized in that** the varnish is a solvent containing varnish comprising an organic solvent medium, at least one film-forming agent, at least one plasticizer, at least one resin, if appropriate a rheology agent, and contains said chestnut extract in a proportion comprised between 0.01% and 1% by weight, preferably comprised between 0.05% and 0.8% by weight, more preferably between 0.1% and 0.6% by weight.

5. The composition according to claim 4 **characterized in that** the organic solvent medium comprises one or a plurality of solvents selected from: short-chain esters with a total of 3 to 8 carbon atoms, such as ethyl acetate, methyl acetate, propyl acetate, isopropyl acetate, butyl acetate, isopentyl acetate, acetate, methoxypropyl, butyl lactate, ketones liquid at room temperature, alcohols such as ethanol, isopropanol, butanol, diacetone alcohol, saturated linear or branched aliphatic hydrocarbons such as alkanes, or unsaturated aliphatic hydrocarbons liquid at room temperature, aromatic hydrocarbons, such as toluene or xylene, and mixtures thereof.

6. The composition according to claim 5, **characterized in that** the organic solvent medium comprises ethyl acetate and/or butyl acetate and/or at least one alcohol selected from ethanol, isopropanol and butanol.

7. The composition according to any of claims 2 and 3, **characterized in that** the varnish is an aqueous-based varnish comprising an aqueous solvent medium, at least one film-forming agent, at least one plasticizer, at least one resin, optionally a film-forming agent a rheology agent and contains said chestnut extract in a proportion comprised between 0.01% and 10% by weight, preferably comprised between 0.05% and 8% by weight, more preferably comprised between 0.1% and 6% by weight.

8. The composition according to any of the preceding claims, **characterized in that** the varnish comprises at least one cellulose film-forming agent chosen from nitrocellulose, carboxymethylcellulose, hydroxyethylcellulose, hydroxypropylcellulose, cellulose acetate butyrate and/or cellulose acetate propionate.

9. The composition according to any of the preceding claims, **characterized in that** the varnish comprises one or a plurality of monomers or oligomers based on (meth)acrylate, urethane, carbamate, isophorones and/or isocyanides, and one or a plurality of photoinitiators.

10. The composition according to any of claims 4 to 9, **characterized in that** the varnish comprises at least one resin chosen from polyvinyl butyral resins, shellac resins, elemi resins, alkyd resins, polyester resins; epoxytosylamide resins; epoxy resins; acrylate resins; styrene-acrylate resins; polyurethane resins; sucrose benzoate; tosylamide/formaldehyde resins, toluenesulfonamide/formaldehyde resins, or polymers and/or copolymers of said different resins.

11. The composition according to any of the preceding claims, **characterized in that** the varnish is a tinted varnish containing at least one coloring substance, pigments, pearlescent agents, flakes, metal particles or a mixture thereof.

12. The composition according to any of the preceding claims **characterized in that** same comprises one or a plurality of nail care products other than the chestnut extract chosen from vitamins A, B, C, E and the derivatives thereof, calcium, silanes, alkylsulfones, amino acids or peptides, in particular the derivatives containing sulfur, and salts thereof, hydrolyzed keratin, alpha-hydroxy acids, urea and the derivatives thereof, oils of vegetable origin, plant extracts other than chestnut extract, and a combination thereof.

13. A use of the cosmetic composition according to any of the preceding claims for improving the surface state of the nail.

14. The use of the composition according to claim 13 for nourishing and moisturizing the nail.
